(19)

**Europäisches Patentamt**

**European Patent Office**

**Office européen des brevets**

(11) **EP 4 769 416 A1**

(12) **EUROPEAN PATENT APPLICATION**

(43) Date of publication:
**01.07.2026 Bulletin 2026/27**

(21) Application number: **24383487.6**

(22) Date of filing: **31.12.2024**

(51) International Patent Classification (IPC):
***G16B 20/30*** (2019.01)       ***G16B 30/10*** (2019.01)
***G16B 40/20*** (2019.01)

(52) Cooperative Patent Classification (CPC):
**G16B 40/20; G16B 20/30; G16B 30/10;**
G16B 35/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Multiverse Computing, S.L.**
**20014 Donostia - San Sebastian, Guipuzcoa (ES)**
• **Asociación Centro de Investigación Cooperativa
en Biomateriales - CIC biomaGUNE**
**20014 San Sebastián (ES)**

(72) Inventors:
• **FLECHAS MANRIQUE, Natalia**
**20014 Donostia-San Sebastián Gipuzkoa (ES)**
• **MARTINEZ DIEZ, Alberto**
**20014 Donostia-San Sebastián Gipuzkoa (ES)**

• **ESPINOSA PORTALÉS, Llorenç**
**20014 Donostia-San Sebastián Gipuzkoa (ES)**
• **ANDREA, Luc**
**92800 PUTEAUX (FR)**
• **MUGEL, Samuel**
**Toronto, M5C 2T2 (CA)**
• **ORÚS, Román**
**20014 Donostia-San Sebastián Gipuzkoa (ES)**
• **LÓPEZ CORTAJARENA, Aitziber**
**20014 SAN SEBASTIÁN (ES)**
• **LÓPEZ MARTÍNEZ, Elena**
**20014 SAN SEBASTIÁN (ES)**
• **MANTECA, Aitor**
**20014 SAN SEBASTIÁN (ES)**

(74) Representative: **Balder IP Law, S.L.**
**Paseo de la Castellana 93
5ª planta
28046 Madrid (ES)**

(54) **A METHOD FOR GENERATING CANDIDATES OF AMINO ACID SEQUENCES OF EPITOPES**

(57)     The invention relates to a method for generating candidates of amino acid sequences of epitopes, the method comprising executing, in one or more processing devices, a neural network for generating candidates of sequences of epitopes, the neural network being a result of fine-tuning a neural network trained for generating amino acid sequences, wherein the training has been performed with data of amino acid sequences comprising amino acid sequences of non-epitopes, and the fine-tuning has been performed with data of amino acid sequences of epitopes.

EP 4 769 416 A1

## Description

### TECHNICAL FIELD

The present invention is encompassed within the field of methods for generating candidates of amino acid sequences of epitopes.

### BACKGROUND

The immune system holds a central significance in human health, serving the crucial function of safeguarding the body against infections. Immune response involves antigen-antibody interaction, a non-covalent chemical interaction between the antibody or immunoglobulin, -a protein belonging to the immune system-, and the antigen, -the molecule that triggers the reaction and, under frequent conditions, belongs to a pathogen or an internal threat, such as a tumor-.From the chemical point of view, proteins consist of chains of smaller molecules called amino acids, which are joint with covalent bonds and form the primary structure of the protein. Antigen regions that specifically bind to the recognition site of an antibody are called epitopes. A protein may contain more than one epitope and the epitope-antibody relationship is not one to one, since a given epitope may bind to more than one antibody and vice versa.

Given the role of epitopes in triggering an immune response, the prediction of antibody-antigen binding and the identification of epitopes is a crucial goal in immunology. However, testing candidate sequences can be a strenuous task. Restricting to sequences with n amino acids, there are $20^n$ different possibilities, out of reach for any experimental facility when the number of amino acids if of about 6 or more, even for those facilities equipped with high throughput screening technologies. Hence, it is desirable to predict amino acid sequences having a relatively high probability of being epitopes.

### SUMMARY

A first aspect of the disclosure relates to a method for generating candidates of amino acid sequences of epitopes, the method comprising executing, in one or more processing devices, a neural network for generating candidates of sequences of epitopes, the neural network being a result of fine-tuning a neural network trained for generating amino acid sequences, wherein the training has been performed with data of amino acid sequences comprising amino acid sequences of non-epitopes, and the fine-tuning has been performed with data of amino acid sequences of epitopes.

In some embodiments, the amino acid sequences used in the training of the neural network comprise amino acid sequences of epitopes. Thereby, the training data may include data of amino acid sequences of epitopes although it is not required that the training data comprises amino acid sequences of epitopes. It may be advantageous that the training data comprises a broad variety of types of amino acid sequences to cause that the neural network learns patterns that affect to amino acid sequences in general.

In some embodiments, the training is unsupervised. For example, the neural network to be trained and the training may be as disclosed in "Ferruz, N., Schmidt, S. & Höcker, B. ProtGPT2 is a deep unsupervised language model for protein design. Nat Commun 13, 4348 (2022). https://doi.org/10.1038/s41467-022-32007-7",

The fine-tuning is performed subsequently to the training. In general, the fine-tuning is a type of training which involves smaller adjustments of the weight parameters of the neural network than the adjustments of the weight parameters performed in the previous training (e.g., of a previous training of a pseudo-randomly initialized neural network).

In the fine-tuning, weight parameters of the neural network are adjusted to optimize the performance of a particular task performed by the neural network. The neural network resulting from the previous training is fine-tuned with data of amino acid sequences of only epitopes. In particular, the neural network may be fine-tuned to perform the task of predicting a subsequent amino acid or portion of amino acid sequence based on the amino acids previously predicted in that sequence (i.e., including based on the position, in the sequence, of each one of the amino acids previously predicted).

In some embodiments, the method is for generating candidates of amino acid sequences of linear epitopes.

In some embodiments, the data of amino acid sequences of epitopes used in the fine-tuning are amino acid sequences of linear epitopes.

In some embodiments, all amino acid sequences of the data used in the fine-tuning have less than twelve residues. Thereby, the probability that each amino acid sequence of the data used in the fine-tuning refers to a linear epitope, instead of to a conformational epitope, is increased.

In some embodiments, the method comprises classifying, by executing at least one classifier in one or more processing devices, the candidates as candidates with higher likelihood of being epitopes and candidates with lower likelihood of being epitopes, the at least one classifier having been trained with data of amino acid sequences of epitopes and of amino acid sequences of non-epitopes to classify amino acid sequences of epitopes as amino acid sequences with higher likelihood of being epitopes and amino acid sequences of non-epitopes as amino acid sequences with lower likelihood of being epitopes.

Because the fine tuning is performed with data of sequences of epitopes, the neural network has not been fine-tuned to detect amino acid sequences of non-epitopes. Therefore, to eliminate those candidates which have a lower probability of being epitopes, classifiers which have been trained with data of amino acid sequences of non-epitopes can be used to process the candidates generated by the fine-tuned neural network.

In some embodiments, the at least one classifier comprises a classifier of viral epitopes and a classifier of bacterial epitopes, and the step of classifying comprises classifying the generated candidates in at least one of the classifier of viral epitopes and the classifier of bacterial epitopes; wherein the classifier of viral epitopes is configured to classify the candidates as candidates with higher likelihood of being viral epitopes and candidates with lower likelihood of being viral epitopes, and the classifier of bacterial epitopes is configured to classify the candidates as candidates with higher likelihood of being bacterial epitopes and candidates with lower likelihood of being bacterial epitopes.

Using classifiers of viral epitopes and of bacterial epitopes is advantageous because most of known epitopes are at least one of bacterial and viral, so that it is expected that most of the candidates generated by the neural network are at least one of bacterial and viral. In addition, training said classifiers, compared to training classifiers with other types of amino acid sequences which are scarcer than bacterial and viral epitopes, may result in better training of the classifiers because of the larger amount of training data and hence in enhanced classification of amino acid sequences.

The classifier of bacterial epitopes is trained with data of amino acid sequences of bacterial epitopes and with data of amino acid sequences of non-bacterial epitopes (i.e., epitopes which are non-bacterial) to classify the amino acid sequences of bacterial epitopes as epitopes and the amino acid sequences of non-bacterial epitopes as non-epitopes.

The classifier of viral epitopes is trained with data of amino acid sequences of viral epitopes and with data of amino acid sequences of non-viral epitopes (i.e., epitopes which are non-viral) to classify the amino acid sequences of viral epitopes as epitopes and the amino acid sequences of non-viral epitopes as non-epitopes.

In some embodiments, a bias of the at least one classifier is adjusted to increase recall at the cost of decreasing at least one of accuracy and precision.

Increasing recall allows maximizing the number of true positives, so that less candidates which have a relatively high probability of being epitopes are wrongly classified by the at least one classifier as non-epitopes (i.e., less false negatives are obtained).

In some embodiments, the bacterial (viral) classifier comprises bacterial (viral) subclassifiers. Each bacterial (viral) subclassifier is configured to classify the candidates as candidates with higher likelihood of being bacterial (viral) epitopes and candidates with lower likelihood of being bacterial (viral) epitopes. The classification provided by the classifier as bacterial (viral) or non-bacterial (non-viral) is based on an aggregation of the classifications provided by the subclassifiers of the classifier. The bias can be the weight given to a classification, by subclassifiers, as bacterial (viral) relative to the weight given to a classification, by subclassifiers, as non-bacterial (non-viral). Thereby, by increasing the weight given to the classifications as bacterial (viral) by the subclassifiers relative to the classifications, by the subclassifiers, as non-bacterial (non-viral) less false negatives are obtained thus increasing recall. For example, the weight given to the classifications as bacterial is higher than 0.5, for example, higher than 0.6 times the weight given to the classifications as non-bacterial. For example, the weight given to the classifications as viral is higher than five, for example, higher than six times the weight given to the classifications as non-viral.

In some embodiments, the subclassifiers are XGBoost classifiers.

The recall of a classifier may be calculated with the following equation:

$$Recall = \frac{True\ Positives}{True\ Positives + False\ Negatives}$$

In some embodiments, the recall of each classifier of the at least one classifier is of at least 60%, for example, of at least 70%.

The accuracy of a classifier may be calculated with the following equation:

$$Accuracy = \frac{True\ Positives + True\ Negatives}{True\ Positives + True\ Negatives + False\ Positives + False\ Negatives}$$

In some embodiments the accuracy of each classifier of the at least one classifier is of at most 70%, for example, of at most 60%, for example, at most than 50%.

In some embodiments, the accuracy of each classifier of the at least one classifier is of at least 30%, for example, of at least 40%.

The precision of a classifier may be calculated with the following equation:

$$Precision = \frac{True\ Positives}{True\ Positives + False\ Positives}$$

In some embodiments the precision of each classifier of the at least one classifier is of at most 70%, for example, of at most 60%, for example, at most than 50%.

In some embodiments, the accuracy of each classifier of the at least one classifier is of at least 10%, for example, of at least 20%.

In some embodiments, the method comprises synthesizing epitope candidates having sequences equal to the candidate sequences.

In some embodiments, the step of synthesizing epitope candidates comprises synthesizing the epitope candidates having sequences equal to the candidate sequences classified by the at least one classifier as having higher likelihood of being an epitope, and not synthesizing the epitope candidates having sequences equal to the candidate sequences classified by the at least one classifier as having lower likelihood of being an epitope.

In some embodiments, the synthesized epitope candidates are tested with antibodies to determine, based on epitope-antibody recognition, if the candidates are epitopes; preferably wherein the determination is based on readout (e.g., fluorescent readout) of epitope-antibody recognition. Thereby, each candidate is tested to determine whether the candidate is an epitope.

In some embodiments, the neural network is autoregressive. For example, the neural network is a generative pre-trained transformer, for example, generative pre-trained transformer 2 (i.e., GPT-2).

In some embodiments, hyperparameters of the fine-tuned neural network comprise temperature and repetition penalty.

In some embodiments, the neural network comprises transformer blocks in series, the initial transformer block is configured to receive embeddings of amino acid sequences, and each transformer block comprises an attention module and a feed forward module.

In some embodiments, the attention module is a multi-head attention module.

In some embodiments, the embeddings are a combination of token embeddings and positional embeddings.

In some embodiments, each transformer block comprises first and second modules of layer normalization, first and second dropout modules and first and second shortcut connections; wherein an output of the first module of layer normalization is connected to an input of the attention module, an output of the attention module is connected to an input of the first dropout module, the first shortcut connection combines the input of the first layer normalization with the output of the first dropout module, an input of the second module of layer normalization being configured to receive said combination; an output of the second layer normalization being connected to the input of the feed forward module, an output of the feed forward module being connected to an input of the second dropout module, and the second shortcut connection combines the input of the second module of layer normalization with the output of the second dropout layer.

In some embodiments, the feed forward module comprises a first linear layer, an activation layer and a second linear layer, wherein an output of the first linear layer is connected to an input of the activation layer, and an output of the activation layer is connected to an input of the second linear layer; wherein the activation layer is preferably a gaussian error linear unit.

In some embodiments, the fine-tuning is performed by minimizing the following cost function:

$$l_{CLM} = -\sum_{k=1}^{D} log\ p_\theta\left(\omega_i^k | \omega_{<i}^k\right)$$

wherein:

- $p_\theta\left(\omega_i^k | \omega_{<i}^k\right)$ is the probability obtained by the neural network for the actual token value $\omega_i^k$ given the values of the previous tokens of the same amino acid sequence;

- $\omega_i^k$ is the actual token value in the i-th position of the k-th sequence of the data of amino acid sequences of epitopes; and

- $D$ is the total number of sequences in the data of amino acid sequences of epitopes.

A second aspect of the disclosure relates to a computer program comprising instructions which, when the program is executed by a computer, cause the computer to carry out the method of the first aspect of the disclosure.

A third aspect of the disclosure relates to one or more processing devices configured for executing the method of the first aspect of the disclosure.

Similar advantages as those described for the first aspect of the disclosure may also be applicable to the second and third aspects of the disclosure.

**BRIEF DESCRIPTION OF THE DRAWINGS**

To complete the description and to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:

Figure 1 shows a method for training and fine-tuning a neural network in accordance with embodiments.

Figure 2 shows a transformer block in accordance with embodiments.

Figure 3 shows a feed forward module in accordance with embodiments.

Figure 4 shows a method in accordance with embodiments.

Figure 5 shows distribution of sequence lengths in accordance with embodiments.

Figure 6 shows relative entropies of sequences with nine residues in accordance with embodiments.

Figure 7 shows global propensities of sequences with nine residues in accordance with embodiments.

Figure 8 shows mutual information between pairs of amino acid distributions at two residues positions in accordance with embodiments.

**DETAILED DESCRIPTION**

The following description is not to be taken in a limiting sense but is given solely for the purpose of describing the broad principles of the invention. Embodiments of the invention will be described by way of example, with reference to the above-mentioned drawings.

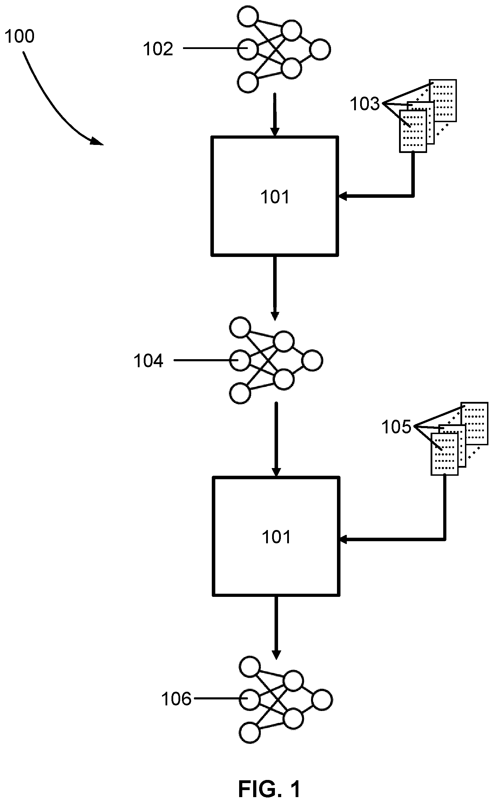

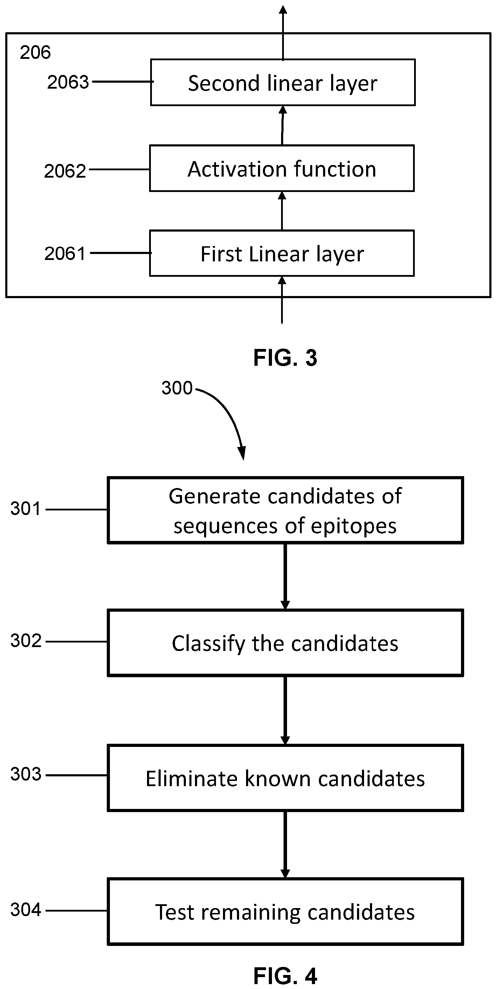

Figure 1 shows a method 100 for training and fine-tuning a neural network and Figure 4 shows a method 300 which involves generating candidates of sequences of epitopes with the neural network resulting from method 100. Method 100 and method 300 are performed in one or more processing devices. The one or more processing devices 101 are, for example, at least one of: at least one CPU, at least one GPU, at least one FPGA, at least one ASIC, at least one personal computer and at least one laptop.

As shown in figure 1, method 100 comprises training, in one or more processing devices 101, a neural network 102 with training data 103.

The training data 103 comprises amino acid sequences of non-epitopes. An example of training data are amino acid sequences included in the Uniref50 version 2021_04. For example, the training data may comprise about 50 million amino acid sequences.

Each amino acid sequence comprises characters wherein each character corresponds to an amino acid.

To be able to use the training data 103 in the training of the neural network 102, the training data 103 is converted to numerical vectors, commonly known as embeddings.

To obtain token embeddings, the training data 103 is tokenized. Each token embedding is a numerical vector representing a different character or sequence of characters (i.e., a portion of an amino acid sequence) of an amino acid sequence. The token embeddings are obtained, for example, by Byte Pair Encoding (BPE) tokenization or by one-hot tokenization.

To further enhance the training of the neural network, positional embeddings can be used in addition to token embeddings. A positional embedding is a numerical vector representing a position, in an amino acid sequence, of a portion of an amino acid sequence (i.e., of a character or sequence of characters). The positional embedding can be independent of the particular amino acid sequence in the position represented by the positional embedding. The positional embeddings may represent absolute positions in an amino acid sequence (i.e., first position of the amino acid sequence, second position of the amino acid sequence, third position of the amino acid sequence, etc.) or relative positions in an amino acid sequence.

The one or more processing devices 101 process the training data 103 to convert each amino acid sequence of the training data 103 in the embeddings. The embeddings are, for example, token embeddings or embeddings resulting from combining (e.g., summing) token embeddings and positional embeddings. The embeddings are stored in, e.g., a non-transitory data storage for subsequent use in the training of the neural network 102.

The number of token embeddings is, for example, of about fifty thousand. An average of four amino acids may be represented by each token embedding.

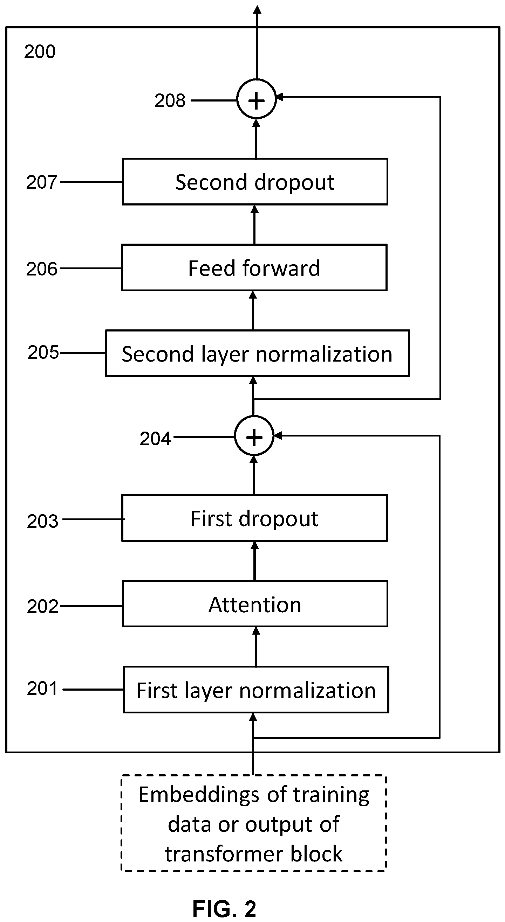

The neural network 102 is, for example, a transformer, for example, a generative pre-trained transformer (GPT), for example, generative pre-trained transformer 2 (GPT-2). The neural network 102 comprises, for example, transformer blocks in series. An example of a transformer block 200 is shown in Figure 2.

A tensor comprising embeddings of the training data or the output of a previous transformer block 200 is input to a module of first layer normalization 201. In the first layer normalization 201, the one or more processing devices adjust values of the components of the input tensor to have a mean of zero and a variance of one. This layer normalization allows enhancing the training of the neural network (e.g., contributes to minimizing the vanishing and/or exploding gradient problem). Obviously, if the transformer block 200 is the initial transformer block of the neural network (i.e., the transformer block to which embeddings of the training data are input) and the embeddings of the training data have previously been normalized, it is not required that the transformer block comprises the first layer normalization 201.

The output of the first layer normalization 201 is inputted to an attention module 202 (e.g., a multi-head attention module). In the attention module 202, the one or more processing devices 101, process the input to combine a latest embedding (i.e., the embedding immediately previous to the embedding to be predicted by the neural network) with previous embeddings of the same amino acid sequence. Thereby, the prediction of the neural network does not only depend on the value of the latest embedding but also in its context, in particular, on the values of previous embeddings of the same amino acid sequence.

The first dropout module 203 and second dropout module 207 are for regularizing the model and preventing/-minimizing overfitting. The first dropout module 203 and second dropout module 207 are used in the training, and, optionally, in the fine-tuning, of the neural network and, in general, are not used once the neural network has been trained and/or fine-tuned. In the dropout modules 203, 207, the outputs of some neurons of the neural network are ignored. In particular, in the first dropout module 203, the outputs of some neurons of the attention module are ignored (e.g., the one or more processing devices 101 set the weight parameters of ignored neurons of the attention module to zero). In the second dropout module 207, the outputs of some neurons of the feed forward module are ignored (e.g., the one or more processing devices 101 set weight parameters of the ignored neurons of the feed forward module to zero).

In the module of first shortcut connection 204, the one or more processing devices 101 sum the output of the first dropout module 203 and the input of the module of first layer normalization 201. The shortcut connections allow minimizing the problem of vanishing gradient descent which appears in deep neural networks.

Similarly to the module of first layer normalization 201, in the module of second layer normalization 205, the one or more processing devices 101 process the input tensor to adjust the values of the tensor to have a mean of zero and a variance of one.

In the feed forward module 206, the one or more processing devices 101 process the output of the second layer normalization 205 applying linear layers and an activation function. An example of a feed forward module is shown in Figure 3. The feed forward module 206 comprises a first linear layer 2061, an activation function 2062 and a second linear layer 2063. In each linear layer the one or more processing devices 101 perform the following operation:

$$Y = XW + B$$

where:

Y is a tensor output by the linear layer;
X is a tensor input to the linear layer;
$W$ is a tensor having as components weight parameters of the linear layer; and
$B$ is a bias tensor.

The tensor outputted by the first linear layer 2061 may have more components (e.g., a higher dimension) than the tensor inputted to the first linear layer 2061.

The activation function is generally a non-linear function, for example, Gaussian Error Linear Unit (GELU), Rectified linear unit (ReLU) or Swish-gated linear unit (SwiGLU). In the activation function, the one or more processing devices 101, may process each component of the input tensor by applying the activation function 2062 to said component. Thereby, the tensor outputted by the activation function 2062 has the same dimensions as the tensor inputted to the activation function 2062.

The tensor outputted by the second linear layer 2063 may have less components (e.g., a lower dimension) than the tensor inputted to the second linear layer 2063.

In the module of second shortcut connection 208, the one or more processing devices 101 sum the output of the second dropout and the input of the first shortcut connection 204, thereby generating the output of the transformer block 200.

The output of the transformer block 200 is the input of a subsequent transformer block, which may have the same architecture as transformer block 200, until reaching the last transformer block of the neural network. The input and output of a transformer block 200 may have the same dimensions.

The one or more processing devices 101 are configured to select the predicted character or sequence of characters based on values of components of the tensor outputted by the last transformer block of the neural network. Said outputted tensor components are commonly known as logits in the field of machine learning. Logits are raw scores generated by the last transformer block. There is a logit associated with each token embedding (i.e., associated with the character or sequence of characters represented by the token embedding), and the value of each logit gives the probability that the character/sequence of characters of the next prediction be the character/sequence of characters represented by the token embedding associated with the respective logit. In general, higher logit values are indicative of higher probability.

In the training, since the next actual embedding of the amino acid sequence is known, a cost function is calculated by comparing, by the one or more processing devices 101, the predicted embedding with the actual embedding, and the values of weight parameters of the neural network are adjusted, by the one or more processing devices 101, to optimize the result of the cost function (e.g., by applying gradient descent). The cost function is, for example,

$$l_{CLM} = -\sum_{k=1}^{D} log\, p_\theta\left(\omega_i^k | \omega_{<i}^k\right)$$

wherein:

- $p_\theta\left(\omega_i^k | \omega_{<i}^k\right)$ is the probability obtained by the neural network for the actual token value $\omega_i^k$ given the values of the previous tokens of the same amino acid sequence;

- $\omega_i^k$ is the actual token value in the i-th position of the k-th sequence of the data of amino acid sequences of epitopes; and

- $D$ is the total number of sequences in the data of amino acid sequences of epitopes used for training the neural network.

Thereby, by optimizing the aforementioned cost function, the probability that the predicted character/character sequence matches the actual character/character sequence is increased. In this way, the neural network 102 is trained with the training data, thus obtaining neural network 104 trained for generating amino acid sequences.

The values of the weight parameters to be optimized may be the values of the weight parameters of the module of feed forward network and the values of weight parameters of the attention module.

The neural network 104 is subsequently fine-tuned to generate candidates of amino acid sequences of epitopes. The fine-tuning may be executed by the same processing device(s) 101 used in the training or by different processing device(s). The execution of the fine-tuned neural network to generate candidates may be performed by the same processing device(s) 101 used in the training and/or in the fine-tuning or by different processing device(s).

To fine-tune the neural network 104, fine-tuning data 105 of amino acid sequences of epitopes is used. In particular, the fine-tuning data may include about 500000 different amino acid sequences of epitopes, for example, of amino acid sequences of epitopes retrieved from "The immune epitope database (IEDB)" (see, e.g., Randi Vita et al. "The immune epitope database (IEDB) 3.0". In: Nucleic Acids Research 43 (2014), pp. D405 - D412. url: https://api. semanticscholar.org/CorpusID:7931138; and see, e.g., Randi Vita et al. "The Immune Epitope Database (IEDB): 2018 update". In: Nucleic Acids Research 47 (2018), pp. D339 -D343. url: https://api.semanticscholar.org/Corpu sID:53026444). Each of the amino acid sequences in the fine-tuning data may have less than twelve residues. Limiting the fine-tuning data to amino acid sequences having less than twelve residues is advantageous because increases the chances that the epitopes in the fine-tuning data are linear epitopes (thereby, decreasing the chances that the epitopes within the fine-tuning data are conformational epitopes). The method of the present disclosure has been found particularly advantageous for generating candidates of amino acid sequences of linear epitopes.

To check that the fine-tuning data is suitable for the fine-tuning, several features of the fine-tuning data may be evaluated. For example, it may be determined that the fine-tuning data is experimentally validated data known to contain the ground-truth. In addition, it may be determined that the fine-tuning data does not contain unwanted biases. Furthermore, it may be checked that the sequence lengths have an appropriate range of values and that no spurious correlations between residues are found, i.e., the mutual information between two residues is much smaller than the entropy of a single residue.

In the fine-tuning, values of the weight parameters of the neural network (e.g., of the weight parameters of the feed forward module and of the weight parameters of the attention module) are optimized to predict the next token embedding. More particularly, as explained above, the neural network generates, for each token embedding, a value indicative of the probability that the character/sequence of characters represented by the respective token embedding is the next prediction.

The fine-tuning can be performed using the same cost function that was used in the training and using the fine-tuning data 105 instead of the training data 103.

As a result of the fine-tuning, the neural network 106 fine-tuned to generate candidates of amino acid sequences of epitopes is obtained. In each execution of the neural network, the neural network predicts the following token embedding of the amino acid sequence based on the previous embeddings predicted by the neural network. Thereby, upon iteratively executing the neural network, a complete amino acid sequence of an epitope candidate is obtained.

When executing the neural network 106 to obtain the epitope candidates, the dropout modules of the neural network are not executed in general because the dropout modules can be merely are used to minimize overfitting in the training and, optionally, in the fine-tuning.

A parameter of repetition penalty and a parameter of temperature of the neural network may be adjusted to increase the number of different candidates generated by the neural network.

Repetition penalty is a parameter which decreases the probability of repeating the character(s) represented by a same token embedding in the same epitope candidate. By increasing the repetition penalty, said probability of repetition is decreased. For example, the repetition penalty may be implemented in those cases in which there is repetition of character(s) by using the following formula:

$$Y_i = \frac{X_i}{RP * n_i}$$

where:

$X_i$ is the value indicative of probability of the i-th token embedding as outputted by the neural network (e.g., $X_i$ is a logit);
$RP$ is the repetition penalty;
$n_i$ is the number of previous appearances of the i-th token embedding in the amino acid sequence; and
$Y_i$ is the updated value indicative of probability of a the i-th token embedding.

In some embodiments, $n_i$ refers to the number of previous appearances of the i-th token embedding in amino acid sequences previously generated by the neural network instead of or in addition to in the same amino acid sequence.

It has been found that repetition penalties higher than 1.2, for example, of at least 2 and at most 3 have lower perplexities than a lower repetition penalty.

In some embodiments, the repetition penalty is not used in the training and/or is not used in the fine-tuning.

In some embodiments, the repetition penalty is used in the generation of candidates of amino acid sequences of epitopes. Thereby, the repetition penalty decreases the probability that a same token is repeated (e.g., repeated in the same amino acid sequence).

The repetition penalty adjusts the probability of selection as the next character sequence of characters based on the quantity of previous appearances of the respective character/sequence of characters. This is done, for example, by adjusting the logits based on the quantity of previous appearances of the respective character/sequence of characters.

The one or more processing devices 101 may apply a function (e.g., a softmax function) to the logits to obtain the probabilities of the predicted portion of amino acid sequence (i.e., the predicted character/sequence of characters). The probability of each token embedding is given by a relationship between the value outputted by the neural network associated with the token embedding (e.g., the value of the logit associated with the token embedding) and the values, outputted by the neural network, associated with the rest of token embeddings of the possible predicted portion of amino acid sequence. The one or more processing devices 101 base the selection of the prediction on the probabilities of the token embeddings.

For example, the one or more processing devices 101 may be configured to assign a selection probability different to zero to all the token embeddings for which the calculated probability according to the values outputted by the last transformer block is not zero. Alternatively, the selection probability different than zero may be assigned only to a determined number k of token embeddings having the top probabilities according to the values outputted in the last transformer block (e.g., to the k logits having the top values) instead of to all the token embeddings.

A relationship between the selection probability and the calculated probability can be adjusted with a parameter known as temperature in the field of machine learning. In general, lower temperature values are indicative of a more uniform distribution of selection probabilities. The temperature may be applied in the following manner to the values of all the components V indicative of a probability that a token embedding is the prediction:

$$\frac{V}{T}$$

where:

V: is a value of a tensor component outputted by the last transformer block and is indicative of a probability of a particular token embedding being the prediction (e.g., V is a logit); and
T: is the temperature.

In some embodiments, the temperature value is one. Temperatures different than one have shown to produce similar results, in terms of the probability of the candidates to be actual epitopes, as a temperature value of one. Therefore, selecting a temperature value of one is advantageous for decreasing the processing load without significantly affecting the quality of the obtained candidates.

By executing, in one or more processing devices 101, the neural network 106, candidates of amino acid sequences of epitopes are generated 301. Out of 200000 generated candidates, it has been determined that 192222 candidates are different and not included in the training data.

The statistical data of the candidates generated with the neural network is consistent with statistical data of known epitopes (notably, in the case of linear epitopes). In particular, the fine-tuned neural network is able to capture features present in the fine-tuning data that are qualitatively understood to characterize epitopes, for example, large relative entropy, small Shannon entropy in the last residue position and lower propensity of cysteine. In addition, no correlations have been found between pairs of residue positions for a given sequence length, provided the number of generated sequences for that length is sufficiently large. This is a positive sign that the model is not adding spurious correlations between amino acids. Another positive sign is that the cost function evaluated on a test set converged after just a few epochs.

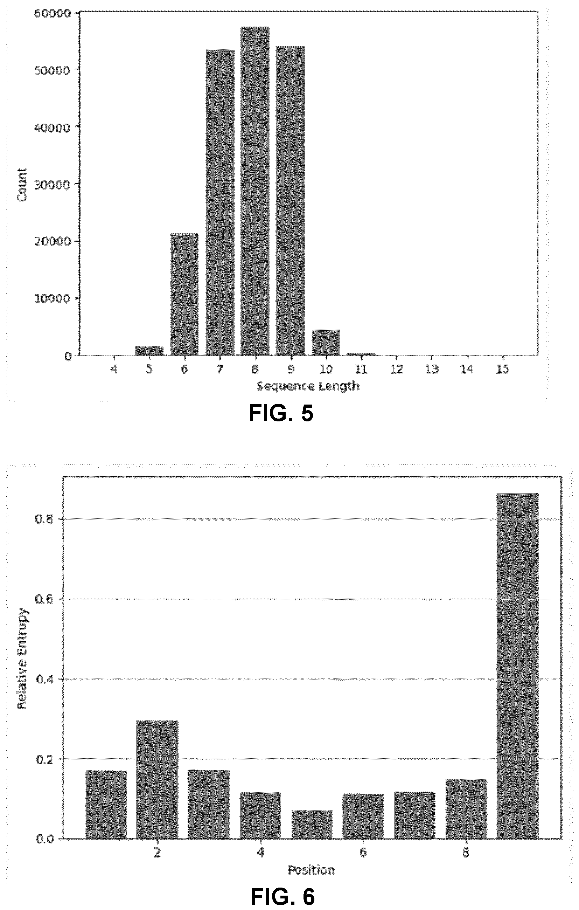

Some statistical details of the generated candidates is shown in Figures 5 to 8 and explained below.

Figure 5 shows distribution of sequence lengths.

Figure 6 shows relative entropies of sequences with nine residues. The relative entropy is between the observed probability distribution of amino acids at each residue position and that of the human proteome. For two discrete probability distributions $P$ and $Q$ defined on a sample space X, the relative entropy is defined as:

$$S_{P||Q} = \sum_{x \epsilon X} P(x) \log\left(\frac{P(x)}{Q(x)}\right)$$

which can be understood as a measure of how different both distributions are.

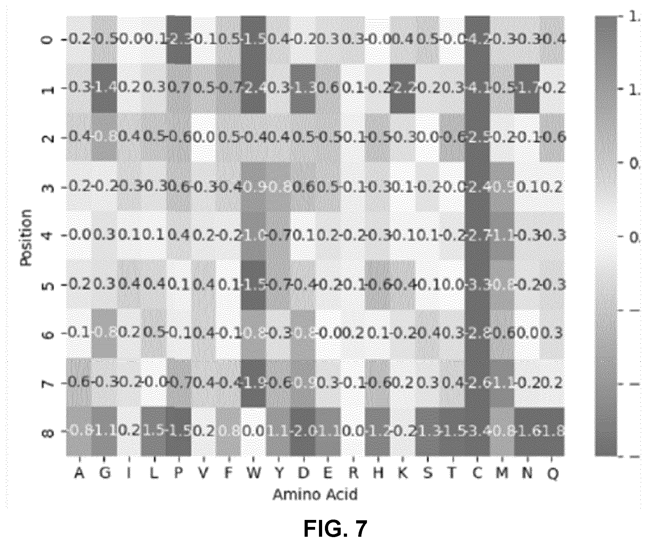

Figure 7 shows global propensities of sequences with nine residues, the global propensity being of amino acids at each residue position. The global propensity of amino acid $X$ at position $y$ in a sample with estimated probability $p_{X,y}$ and a reference probability in the human proteome $q_{X,y}$ is defined as the following ratio:

$$propensity_{X,y} = \frac{p_{X,y}}{q_{X,y}}$$

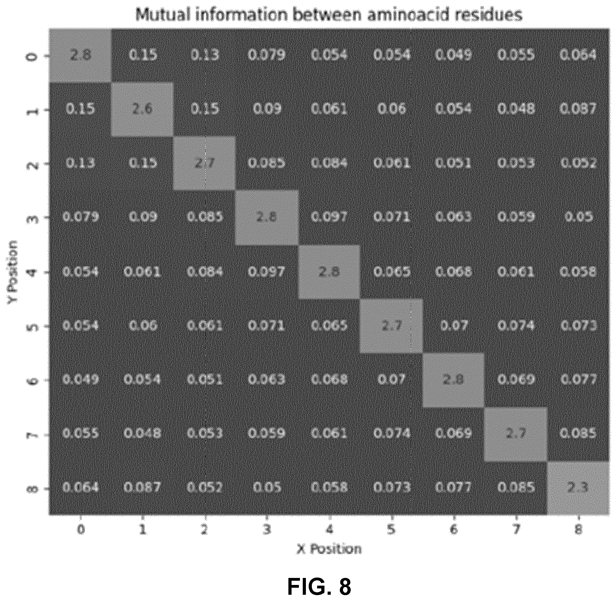

Figure 8 shows mutual information between pairs of amino acid distributions at two residues positions. For two discrete probability distributions $P$ and $Q$ defined on a sample space X, the relative entropy is defined as:

$$I_{P,Q} = S_P + S_Q - S_{P,Q}$$

where $S_{P,Q}$ is the Shannon entropy of the corresponding joint probability distribution. Note that if $P = Q$, then $I_{P,Q} = 2S_P$. Shannon entropy of amino acid distribution at each residue position may be defined, for discrete probability $P$ on a sample space $X$, in the following manner:

$$S_P = -\sum_{x \in X} P(x) \log P(x)$$

which can be understood as an inverse measure of information or difference with an information-less random uniform distribution.

The generated amino acid sequences of epitopes are input to a classifier of viral epitopes and to a classifier of bacterial epitopes.

The classifier of viral epitopes has been trained to classify amino acid sequences as viral epitopes or non-viral epitopes. The classifier of bacterial epitopes has been trained to classify amino acid sequences as bacterial epitopes or non-bacterial epitopes. The training of the classifiers has been performed, in one or more processing devices 101, with positive data (i.e., amino acid sequences of viral epitopes in the case of the classifier of viral epitopes; and amino acid sequences of bacterial epitopes in the case of the classifier of bacterial epitopes) and with negative data (i.e., amino acid sequences of non-viral epitopes in the case of the classifier of viral epitopes; and amino acid sequences of non-bacterial epitopes in the case of the classifier of bacterial epitopes).

The classifiers may be XGBoost classifiers. A training of the classifiers may be performed by adjusting weight parameters of the classifiers to optimize a cost function, e.g., by increasing a number of amino acid sequences of training data correctly classified by the classifiers.

The classifiers may classify each generated candidate based on the values of all the components of the vector defining the generated candidate. It has been shown that classifying a different portion of the vector with each subclassifier of the respective classifier provides similar results as classifying the whole vector in each subclassifier. For example, if the candidate is represented with a vector of 1024 components, a classifier comprising eleven subclassifiers may be configured so that components 1 to 100 of the vector (i.e., the first one hundred components of the vector) are input to a first subclassifier of the eleven subclassifiers, components 101 to 200 of the vector are input to a second subclassifier of the eleven subclassifiers, components 201 to 300 of the vector are input to a third subclassifier of the eleven subclassifiers, ... and components 1001 to 1024 of the vector are input to a eleventh subclassifier of the eleven subclassifiers. Thereby, to decrease the processing load, each different portion of the candidate may be classified by each subclassifier as having a higher probability of belonging to an epitope (e.g., to a viral epitope and/or to a bacterial epitope) or as having a lower probability of belonging to an epitope (e.g., to a viral epitope and/or to a bacterial epitope).. The classification provided by each classifier is based on an aggregation of the classifications provided by the subclassifiers of the respective classifier.

For the same reasons, the training of the classifiers may be performed with different portions of each amino acid sequence of the training data. In particular, each subclassifier may have been trained to process a portion of the vector of the candidate, the portion having a determined location. For example, a first subclassifier may be trained to classify a portion encompassing components 1 to 100 of the vector of the candidate, a second subclassifier may be trained to classify a portion encompassing components 101 to 200 of the vector of the candidate, ..., and an eleventh subclassifier may be trained to classify a portion encompassing components 1001 to 1024 of the vector of the candidate. An example of statistical parameters of the classifiers is shown in Table 1 below:

*Table 1*

| Classifier | Accuracy | Recall | Precision | True Neg | False Pos | False Neg | True Pos |
|------------|----------|--------|-----------|----------|-----------|-----------|----------|
| Bacterial | 0.493 | 0.795 | 0.209 | 0.3675 | 0.4746 | 0.0323 | 0.1254 |
| Viral | 0.485 | 0.818 | 0.415 | 0.1699 | 0.4443 | 0.0701 | 0.3156 |

Only the candidates classified 302 as at least one of viral epitopes by the classifier of viral epitopes and bacterial epitopes by the classifier of bacterial epitopes proceed to the next stage of method 300. Thereby the candidates which are not classified as viral nor as bacterial epitopes are eliminated.

The candidates that proceed to the next stage, but which are known to be epitopes are eliminated 303. The remaining candidates are synthesized and tested to determine whether they are epitopes.

The synthesized candidates may be screened using a system built on an inverted microscope with an optical setup that may provide the ability to visualize the experiment in real time. In addition, one or more processing devices may be configured operate a drop detector. Upon detecting a drop by the drop detector, an actuation signal is sent, by the one or more processing devices, to a deflector arranged in a path of the drop to deflect the drop to a desired channel for subsequent collection and sequencing. To enable fluorescence readout of epitope-antibody recognition, the synthesized epitopes may be fused to a fluorescent protein (e.g., to green fluorescent protein (GFP)). Excitation (488 nm) and emission (523 nm) peaks of the GFP make GFP suitable for detection with a blue laser (473 nm) and a photomultiplier tube (PMT) with bandpass filters in the range of 525 ± 25 nm. The microfluidics platform is based on differences in the fluorescence anisotropy of the system. Fluorescence polarization is the phenomenon where the light emitted by a fluorophore has different intensities along the polarization axes. If the fluorophore binds to a relatively large molecule compared to its size, the rotational diffusion coefficient changes significantly, resulting in a change in anisotropy. In this case, the antibodies are significantly larger than GFP, therefore the fluorescence anisotropy of antibody-bound GFP compared to the unbound state is to be observed.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

**Claims**

1. A method for generating candidates of amino acid sequences of epitopes, the method comprising executing, in one or more processing devices, a neural network for generating candidates of sequences of epitopes, the neural network being a result of fine-tuning a neural network trained for generating amino acid sequences, wherein the training has been performed with data of amino acid sequences comprising amino acid sequences of non-epitopes, and the fine-tuning has been performed with data of amino acid sequences of epitopes.

2. The method of claim 1, comprising classifying, by executing at least one classifier in one or more processing devices, the candidates as candidates with higher likelihood of being epitopes and candidates with lower likelihood of being epitopes, the at least one classifier having been trained with data of amino acid sequences of epitopes and of amino acid sequences of non-epitopes to classify amino acid sequences of epitopes as amino acid sequences with higher likelihood of being epitopes and amino acid sequences of non-epitopes as amino acid sequences with lower likelihood of being epitopes.

3. The method of claim 2, wherein the at least one classifier comprises a classifier of viral epitopes and a classifier of bacterial epitopes, and the step of classifying comprises classifying the generated candidates in at least one of the classifier of viral epitopes and the classifier of bacterial epitopes; wherein the classifier of viral epitopes is configured to classify the candidates as candidates with higher likelihood of being viral epitopes and candidates with lower likelihood of being viral epitopes, and the classifier of bacterial epitopes is configured to classify the candidates as candidates with higher likelihood of being bacterial epitopes and candidates with lower likelihood of being bacterial epitopes.

4. The method of any one of claims 2 and 3, wherein a bias of the at least one classifier is adjusted to increase recall at the cost of decreasing at least one of accuracy and precision.

5. The method of any one of the previous claims, comprising synthesizing epitope candidates having sequences equal to the candidate sequences.

6. The method of claim 5 when depending on claim 2, 3 or 4, wherein the step of synthesizing epitope candidates comprises synthesizing the epitope candidates having sequences equal to the candidate sequences classified by the at least one classifier as having higher likelihood of being an epitope, and not synthesizing the epitope candidates having sequences equal to the candidate sequences classified by the at least one classifier as having lower likelihood of being an epitope.

7. The method of any one of the preceding claims, wherein the neural network is autoregressive.

8. The method of any one of the preceding claims, wherein hyperparameters of the fine-tuned neural network comprise temperature and repetition penalty.

9. The method of any one of the previous claims, wherein the neural network comprises transformer blocks in series, the initial transformer block is configured to receive embeddings of amino acid sequences, and each transformer block comprises an attention module and a feed forward module.

10. The method of claim 9, wherein each transformer block comprises first and second modules of layer normalization, first and second dropout modules and first and second shortcut connections; wherein an output of the first module of layer normalization is connected to an input of the attention module, an output of the attention module is connected to an input of the first dropout module, the first shortcut connection combines the input of the first layer normalization with the output of the first dropout module, an input of the second module of layer normalization being configured to receive said combination; an output of the second layer normalization being connected to the input of the feed forward module, an output of the feed forward module being connected to an input of the second dropout module, and the second shortcut connection combines the input of the second module of layer normalization with the output of the second dropout layer.

11. The method of claim 10, wherein the feed forward module comprises a first linear layer, an activation layer and a second linear layer, wherein an output of the first linear layer is connected with an input of the activation layer, and an output of the activation layer is connected with an input of the second linear layer; wherein the activation layer is preferably a gaussian error linear unit.

12. The method of any one of the previous claims, wherein the fine-tuning is performed by minimizing the following cost function:

$$l_{CLM} = - \sum_{k=1}^{D} log \, p_\theta \left( \omega_i^k | \omega_{<i}^k \right)$$

wherein:

- $p_\theta \left( \omega_i^k | \omega_{<i}^k \right)$ is the probability obtained by the neural network for the actual token value $\omega_i^k$ given the values of the previous tokens of the same amino acid sequence;

- $\omega_i^k$ is the actual token value in the i-th position of the k-th sequence of the data of amino acid sequences of epitopes; and
- $D$ is the total number of sequences in the data of amino acid sequences of epitopes.

13. A computer program comprising instructions which, when the program is executed by a computer, cause the computer to carry out the method of any one of claims 1 to 12.

14. One or more processing devices configured for executing the method of any one of claims 1 to 12.

15. The method of claim 5 or 6 or of any one of claims 7 to 12 when depending on claim 5 or 6, wherein the synthesized epitope candidates are tested with antibodies to determine, based on epitope-antibody recognition, if the candidates are epitopes; preferably wherein the determination is based on fluorescent readout of epitope-antibody recognition.

**FIG. 1**

**FIG. 2**

## 206

2063 — Second linear layer

2062 — Activation function

2061 — First Linear layer

**FIG. 3**

300

301 — Generate candidates of sequences of epitopes

302 — Classify the candidates

303 — Eliminate known candidates

304 — Test remaining candidates

**FIG. 4**

FIG. 5

FIG. 6

**FIG. 7**

FIG. 8

Europäisches
Patentamt

European
Patent Office

Office européen
des brevets

**EUROPEAN SEARCH REPORT**

Application Number

EP 24 38 3487

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/386610 A1 (SINGH GURPREET [US] ET AL) 30 November 2023 (2023-11-30) <br> * claims 1-27 * <br> * paragraph [0001] - paragraph [0002] * <br> * paragraph [0008] - paragraph [0009] * <br> * paragraph [0013] - paragraph [0021] * <br> * paragraph [0032] * <br> * paragraph [0054] * <br> * paragraph [0056] - paragraph [0060] * <br> * paragraph [0062] - paragraph [0076] * <br> * paragraph [0086] * <br> * paragraph [0091] * <br> * paragraph [0141] - paragraph [0158] * <br> * paragraph [0184] * <br> * paragraph [0188] - paragraph [0196] * <br> * paragraph [0198] - paragraph [0208] * <br> * paragraph [0215] - paragraph [0217] * <br> * paragraph [0434] - paragraph [0454] * <br> * paragraph [0480] - paragraph [0488] * <br> * paragraph [0501] - paragraph [0506] * <br> * paragraph [0520] - paragraph [0523] * <br> * Facsimile page 13 * <br> * Facsimile page 17 * <br> ----- | 1-15 | INV. <br> G16B20/30 <br> G16B30/10 <br> G16B40/20 |
| X | US 2024/153590 A1 (ESSAGHIR AHMED [BE] ET AL) 9 May 2024 (2024-05-09) <br> * paragraph [0007] - paragraph [0018] * <br> * paragraph [0026] * <br> * paragraph [0142] * <br> * paragraph [0158] - paragraph [0163] * <br> * paragraph [0193] - paragraph [0201] * <br> * paragraph [0204] - paragraph [0208] * <br> * paragraph [0232] - paragraph [0237] * <br> * paragraph [0240] - paragraph [0241] * <br> * paragraph [0281] - paragraph [0286] * <br> * paragraph [0317] - paragraph [0321] * <br> * paragraph [null] - paragraph [0181] * <br> * Facsimile page 22 * <br> ----- <br> -/-- | 1-15 | TECHNICAL FIELDS SEARCHED (IPC) <br><br> G16B |

The present search report has been drawn up for all claims

| Place of search | Date of completion of the search | Examiner |
|---|---|---|
| Munich | 13 June 2025 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 38 3487

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/192143 A1 (GRITSTONE BIO INC [US]) 19 September 2024 (2024-09-19)<br>* claim 1 *<br>* paragraph [0001] *<br>* paragraph [0003] *<br>----- | 1-15 | |
| X | AU 2023 251 111 A1 (GRITSTONE BIO INC [US]) 21 November 2024 (2024-11-21)<br>* claims 1-17,19 *<br>* paragraph [0004] *<br>* paragraph [0033] - paragraph [0038] *<br>* paragraph [0067] - paragraph [0068] *<br>* paragraph [0089] *<br>* paragraph [0387] - paragraph [0393] *<br>* paragraph [0427] - paragraph [0433] *<br>* paragraph [0474] - paragraph [0476] *<br>* paragraph [0480] - paragraph [0488] *<br>* paragraph [0517] - paragraph [0520] *<br>* paragraph [0523] - paragraph [0531] *<br>----- | 1-15 | |

**TECHNICAL FIELDS SEARCHED (IPC)**

The present search report has been drawn up for all claims

| Place of search | Date of completion of the search | Examiner |
|---|---|---|
| Munich | 13 June 2025 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 38 3487

This annex lists the patent family members relating to the patent documents cited in  the above-mentioned European search report.
The members are as contained in the European Patent Office EDP file on
The European Patent Office is in no way liable for these particulars which are merely given for the purpose of information.

13-06-2025

| Patent document cited in search report | | Publication date | Patent family member(s) | | Publication date |
|---|---|---|---|---|---|
| US 2023386610 | A1 | 30-11-2023 | NONE | | |
| US 2024153590 | A1 | 09-05-2024 | EP | 4302300 A1 | 10-01-2024 |
| | | | US | 2024153590 A1 | 09-05-2024 |
| | | | WO | 2022185179 A1 | 09-09-2022 |
| WO 2024192143 | A1 | 19-09-2024 | NONE | | |
| AU 2023251111 | A1 | 21-11-2024 | AU | 2023251111 A1 | 21-11-2024 |
| | | | CN | 119173271 A | 20-12-2024 |
| | | | EP | 4504254 A1 | 12-02-2025 |
| | | | IL | 316207 A | 01-12-2024 |
| | | | JP | 2025512989 A | 22-04-2025 |
| | | | KR | 20250005198 A | 09-01-2025 |
| | | | WO | 2023196966 A1 | 12-10-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FERRUZ, N.** ; **SCHMIDT, S.** ; **HÖCKER, B.** ProtGPT2 is a deep unsupervised language model for protein design. *Nat Commun*, 2022, vol. 13, 4348, https://doi.org/10.1038/s41467-022-32007-7
- **RANDI VITA et al.** The immune epitope database (IEDB) 3.0. *Nucleic Acids Research*, 2014, vol. 43, D405-D412, https://api.semanticscholar.org/CorpusID:7931138
- **RANDI VITA et al.** The Immune Epitope Database (IEDB): 2018 update. *Nucleic Acids Research*, 2018, vol. 47, D339-D343, https://api.semanticscholar.org/CorpusID:53026444